**Europäisches Patentamt**

**European Patent Office**

**Office européen des brevets**

(11) Veröffentlichungsnummer: **0 012 445**
**B1**

(12) **EUROPÄISCHE PATENTSCHRIFT**

(45) Veröffentlichungstag der Patentschrift:
07.04.82

(21) Anmeldenummer: **79105193.1**

(22) Anmeldetag: **14.12.79**

(51) Int. Cl.³ : **A 61 M 5/14, A 61 M 5/16,**
**A 61 J 1/00**

(54) **Infusionsvorrichtung.**

(30) Priorität: 16.12.78 DE 2854392
26.02.79 DE 2907479

(43) Veröffentlichungstag der Anmeldung:
25.06.80 (Patentblatt 80/13)

(45) Bekanntmachung des Hinweises auf die Patenterteilung: 07.04.82 Patentblatt 82/14

(84) Benannte Vertragsstaaten:
**AT BE CH DE FR GB IT LU NL SE**

(56) Entgegenhaltungen:
FR - A - 2 003 357
US - A - 3 670 728

(73) Patentinhaber: **Bernard, Ingrid**
**Dresdner Strasse 8**
**D-4803 Steinhagen (DE)**

(72) Erfinder: **Kaemmerer, Erich, Dr.**
**Dresdner Strasse 8**
**D-4803 Steinhagen (DE)**

(74) Vertreter: **Patentanwälte Ter Meer-Müller-Steinmeister**
**Artur-Ladebeck-Strasse 51**
**D-4800 Bielefeld 1 (DE)**

Infusions vorrichtung

Die Erfindung betrifft eine Infusionsvorrichtung mit einem eine Öffnung auf der in Gebrauchsstellung unteren Seite aufweisenden, durch einen Verschlußstopfen verschließbaren Behälter zur Aufnahme einer Infusionslösung und einer den Verschlußstopfen durchdringenden Auslauf-Kanüle.

Derartige Infusionsvorrichtungen mit Behältern in der Form von Beuteln oder Flaschen sind in verschiedenen Ausführungsformen bekannt. Die Kanüle wird durch den zumeist aus Gummi oder Kunststoff bestehenden Verschlußstopfen erst bei Inbetriebnahme hindurchgestochen, jedoch soll für die vorliegende Beschreibung von der Betriebstellung des Infusionsgerätes ausgegangen werden, bei der der Behälter mit dem Verschlußstopfen nach unten aufgehängt und die Kanüle in den verschlußstopfen eingestochen ist.

In der medizinischen Praxis ist es vielfach erforderlich, mehrere Präparate gleichzeitig, ggf. auch in unterschiedlichen gegenseitigen Mengenverhältnissen zu verabreichen. Es ist daher häufig erforderlich, mehrere Behälter mit diesen unterschiedlichen Präparaten zu verwenden und diese durch Leitungen in geeigneter Weise zu verbinden und sodann ein Gemisch zu verabreichen. Mehrere Behälter mit entsprechenden Verbindungsleitungen führen jedoch zu einem unübersichtlichen System, das nicht nur unhandlich ist, sondern auch Verwechslungen ermöglicht.

In einigen Fällen ist es möglich, unphysiologische Mischpräparate in ein und demselben Behälter zu liefern, jedoch sind in diesem Falle z.B. physiologisch bedingte Fett- und Glukose-Anteile nicht integrierbar und eine Dosierung der Mengenverhältnisse der Bestandteile ist nicht möglich.

Aus der US-A-3670 728 ist ein Infusionsbehälter mit zwei hintereinander liegenden Kammern bekannt, durch die zwei unterschiedliche Lösungen aufgenommen werden können. Eine Auslauf-Kanüle wird in den Verschluß der einen Kammer eingestochen und durchstößt mit einer Verlängerung eine Trennwand zwischen der Kammern. Dadurch kommt es bereits zu Beginn der Entnahme zu einer vollständigen Vermischung der Inhalte beider Kammern, so daß eine von Fall zu Fall unterschiedliche Dosierung der Mengenanteile nicht möglich ist und unerwünschte Reaktionen zwischen den Mischungsbestandteilen auftreten können.

Der Erfindung liegt die Aufgabe zugrunde, ein Infusionsgerät der eingangs genannten Art zu schaffen, das in einfacher und dosierbarer Weise die gleichzeitige Verabreichung von wenigstens zwei Präparaten gestattet.

Zur Lösung dieser Aufgabe ist das erfindungsgemäße Infusionsgerät dadurch gekennzeichnet, daß der Behälter wenigstens zwei nebeneinander liegende, getrennte Kammern aufweist, daß die Öffnung einer ersten Kammer durch einen Verschlußstopfen mit wenigstens einem inneren Hohlraum verschlossen ist, daß der Hohlraum die Bahn einer Kanüle schneidet und mit einer zweiten Kammer in Verbindung steht und daß diese Kanüle mindestens eine in die erste Kammer und eine in den Hohlraum mündende Öffnung aufweist und als Auslauf-Kanüle ausgebildet ist oder über den Hohlraum mit einer Auslauf-Kanüle in Verbindung steht.

Der erfindungsgemäße Verschlußstopfen, der zugleich die erste Kammer verschießt und mit seinem inneren Hohlraum eine Verbindung zu der zweiten Kammer schafft, eröffnet die Möglichkeit, mit Hilfe einer zwei in Längsabstand liegende Öffnung aufweisenden Kanüle beide Kammern gleichzeitig zu erfassen. Dieser Effekt der Kombination aus Verschlußstopfen und Kanüle kann in verschiedener Weise ausgenutzt werden, wie im Folgenden näher erläutert werden soll, ist jedoch nicht auf zwei Kammern beschränkt, sondern auch auf eine größere Anzahl von Kammern anwendbar.

Bei einer ersten bevorzugten Ausführungsform der Erfindung ist lediglich eine einzige Kanüle vorgesehen, die eine Auslauf-Kanüle darstellt und im Inneren zwei Kanäle aufweist, die jeweils über eine gesonderte Öffnung mit dem Hohlraum bzw. und der ersten Kammer in Verbindung stehen. Diese Kanäle gestatten somit die gleichzeitige Entnahme der Infusionslösung aus beiden Kammern. Zwei zusätzliche Belüftungskanäle in der Kanüle bewirken einen Druckausgleich in beiden Kammern.

Bei einer anderen bevorzugten Ausführungsform der Erfindung ist zusätzlich eine Verbindungs-Kanüle vorgesehen, die die erste Kammer mit dem Hohlraum verbindet und zugleich einen Belüftungskanal für die erste Kammer enthält. Dadurch gelangt die Lösung aus der ersten Kammer über den Hohlraum in die zweite Kammer und vermischt sich an dessen Boden, also unmittelbar oberhalb des entsprechenden Verschlußstopfens mit der anderen Lösung. Diese entstehende Mischung kann durch eine Auslauf-Kanüle mit einer Einlaß-Öffnung abgeführt werden.

Bei einer weiteren Ausführungsform der Erfindung, die gewissermaßen eine Kombination der ersten und zweiten Ausführungsform darstellt, entnimmt eine Auslauf-Kanüle mit zwei Einlaß-Öffnungen Infusionslösungen aus der ersten Kammer und dem Hohlraum, während der Hohlraum mit einem weiteren Hohlraum in einem Verschlußstopfen der zweiten Kammer in Verbindung steht und der letztgenannte Hohlraum über eine Verbindungs-Kanüle mit der zweiten Kammer in Verbindung gesetzt werden kann.

Die Verwendung einer zusätzlichen Verbindungs-Kanüle gestattet es, in die Verbindungsleitung dieser Kanüle ein Dosierventil einzusetzen, so daß die Mengenverhältnisse zwischen beiden Lösungen geregelt werden können, wie es insbesondere bei Lösungen mit unterschiedlicher Viskosität oder unterschiedlichem spezifischem Gewicht notwendig sein kann.

Die Auslauf-Kanüle kann in einem Stück mit einem Mischgefäß verbunden sein, in das die Auslauf-Kanäle münden. Vorzugsweise ragen die Auslauf-Kanäle mit einem als Abtropfende dienenden Rohrabschnitt von oben in das Mischgefäß hinein, und die Abtropfenden sind so dicht beieinander angeordnet, daß sich ein gemeinsamer Tropfen bildet, der zum Boden des Mischgefäßes herabfällt.

Weitere Einzelheiten der Erfindung ergeben sich aus den Unteransprüchen 6 bis 8 und 10.

Im Folgenden werden bevorzugte Ausführungsbeispiele der Erfindung anhand der beigefügten Zeichnung näher erläutert.

Figur 1 ist ein senkrechter, teilweise unterbrochener Schnitt durch einen Behälter mit Verschlußstopfen ;

Figur 2 ist eine Ansicht auf den Behälter ohne Verschlußstopfen von unten in Fig. 1 ;

Figur 3 zeigt eine Draufsicht auf einen beide Kammeröffnungen erfassenden Verschlußstopfen ;

Figur 4 zeigt eine Ansicht auf eine abgewandelte Ausführungsform eines Behälters von unten ;

Figur 5 ist ein senkrechter, teilweise unterbrochener Schnitt entsprechend Fig. 4 ;

Figur 6 zeigt in einer Teildarstellung einen Behälter mit einem Verschlußstopfen und einer Kanüle entsprechend einer Ausführungsform der Erfindung ;

Figur 7 zeigt eine Ausführungsform der Erfindung mit zwei Kanülen ;

Figur 8 zeigt eine weitere Ausführungsform der Erfindung mit zwei Kanülen ;

Figure 9 und 10 zeigen in Draufsicht und Schnitt den Verschlußstopfen gemäß Fig. 8 ;

Figur 11 zeigt eine weitere, teilweise mit derjenigen der Fig. 8 übereinstimmende Ausführungsform ;

Figur 12 ist ein senkrechter Schnitt durch eine Verbindungs-Kanüle gemäß Fig. 11 ;

Figur 13 ist ein Teilschnitt entlang der Linie 13-13 in Fig. 12.

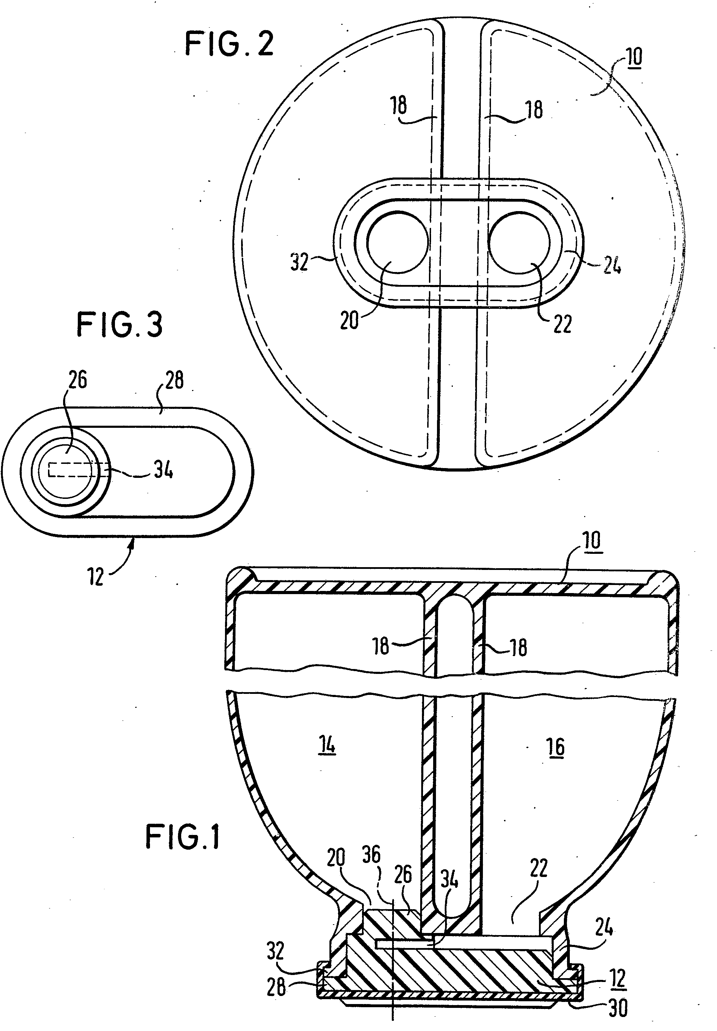

Fig. 1 zeigt einen Behälter 10 mit einem Verschlußstopfen 12 in derjenigen Stellung, die als Betriebsstellung bezeichnet werden kann und bei der sich der Verschlußstopfen an der Unterseite des Behälters befindet. Zum Einfüllen einer Infusionslösung in eine erste und eine zweite Kammer 14 bzw. 16 befindet sich der Behälter 10 in der umgekehrten Position. Zwischen den Kammern 14, 16 befindet sich eine Trennwand 18, die aus herstellungstechnischen Gründen doppelwandig ausgebildet ist. Die Form des Behälters insgesamt ist nicht wesentlich. Der Behälter kann aus Glas oder insbesondere aus Kunststoff bestehen und ggf. auch ein Beutel sein.

Die Kammern 14, 16 weisen je eine Öffnung 20 bzw. 22 auf, die gemäß Fig. 1 und 2 nebeneinander und in einer Ebene liegen und durch einen gemeinsamen, von der Ebene der Öffnungen 20, 22 vorspringenden Rand 24 umgeben sind. Der umlaufende Rand 24 dient zur Aufnahme eines gemeinsamen Verschlußstopfens 12.

Der Verschlußstopfen 12 ist langgestreckt und um die Öffnungen 20 und 22 herum halbkreisförmig abgerundet und weist einen nach außen vorspringenden Flansch 28 auf, der den Rand 24 übergreift. Eine Haltekappe 30 hintergreift einen nach außen vorspringenden Ansatz 32 des Randes 24 und legt den Verschlußstopfen 12 innerhalb des Randes 24 fest.

Zur zuverlässigen Trennung der Kammern 14 und 16 untereinander geht von dem Verschlußstopfen 12 ein zapfenförmiger Ansatz 26 mit im dargestellten Beispiel kreisförmigen Querschnitt aus, der die Öffnung 20 dicht verschließt. Innerhalb dieses zapfenförmigen Ansatzes 26 befindet sich ein Hohlraum 34 in der Form eines Kanals, der von rechts in Fig. 1, d.h. von der Seite der zweiten Kammer 16 aus in den zapfenförmigen Ansatz 26 eintritt.

Ein derartiger Verschlußstopfen bietet die Möglichkeit, im Bereich der Öffnung 20 der ersten Kammer 14, d.h. etwa entlang der strichpunktierten Linie 36, eine Kanüle einzuführen, die, sofern sie in geeignetem Abstand liegende Eintrittsöffnungen aufweist, mit dem Inhalt beider Kammern 14, 16 gleichzeitig in Verbindung tritt, wie später näher erläutert werden soll.

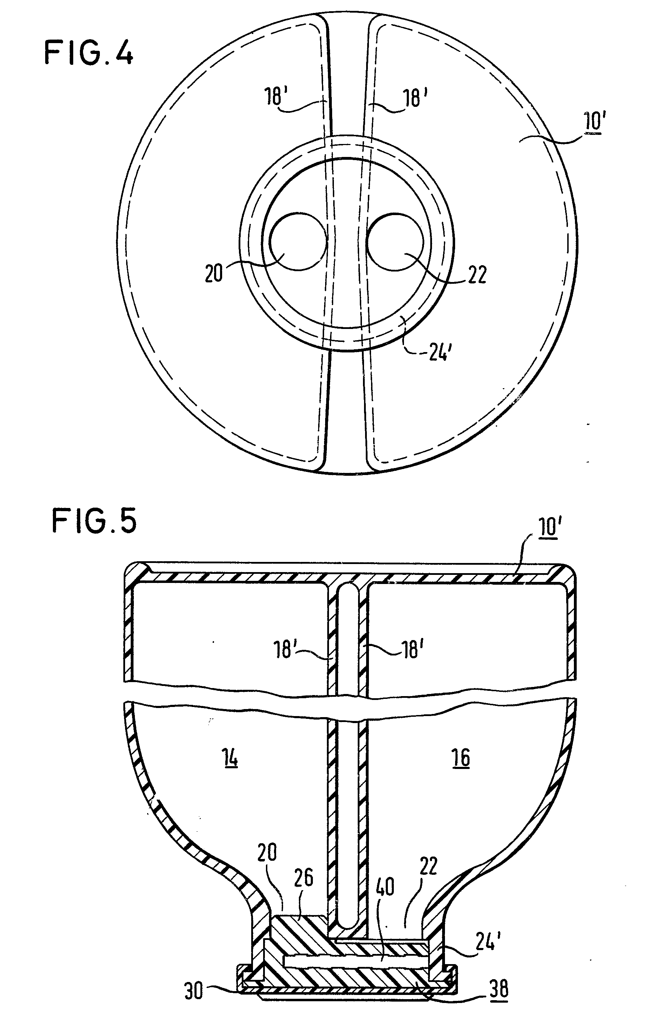

Fig. 4 zeigt eine Ansicht auf einen anderen Behälter 10' von unten. Dieser Behälter 10' unterscheidet sich von dem Behälter 10 der Fig. 2 im wesentlichen dadurch, daß die beiden Wandflächen der Trennwand 18' zwischen den Kammern von der Mitte her nach beiden Seiten leicht schräg auseinanderlaufen, wie es aus herstellungstechnischen Gründen erforderlich sein kann. Im übrigen ist der Rand 24', der die Öffnungen 20 und 22 umgibt, kreisförmig ausgebildet.

Dementsprechend ist auch der in Fig. 5 gezeigte Verschlußstopfen 38 im Umriß kreisförmig. Im übrigen entspricht die Art der Festlegung des Verschlußstopfens 38 innerhalb des randes 24' der bereits im Zusammenhang mit Fig. 1 beschriebenen Lösung, so daß eine erneute Erläuterung nicht notwendig ist. Wie bei der zuvor beschriebenen Ausführungsform weist der Verschlußstopfen 38 unterhalb des zapfenförmigen Ansatzes 26, der in die Öffnung 20 der ersten Kammer 14 eintritt, einen in diesem Falle mit 40 bezeichneten Hohlraum auf, der sich in diesem Falle jedoch nach rechts in Fig. 5 bis zu dem Rand 24' erstreckt und nicht unmittelbar in die zweite Kammer 16 mündet. Auf die Anwendung dieses Verschlußstopfens 38 soll ebenfalls später näher eingegangen werden.

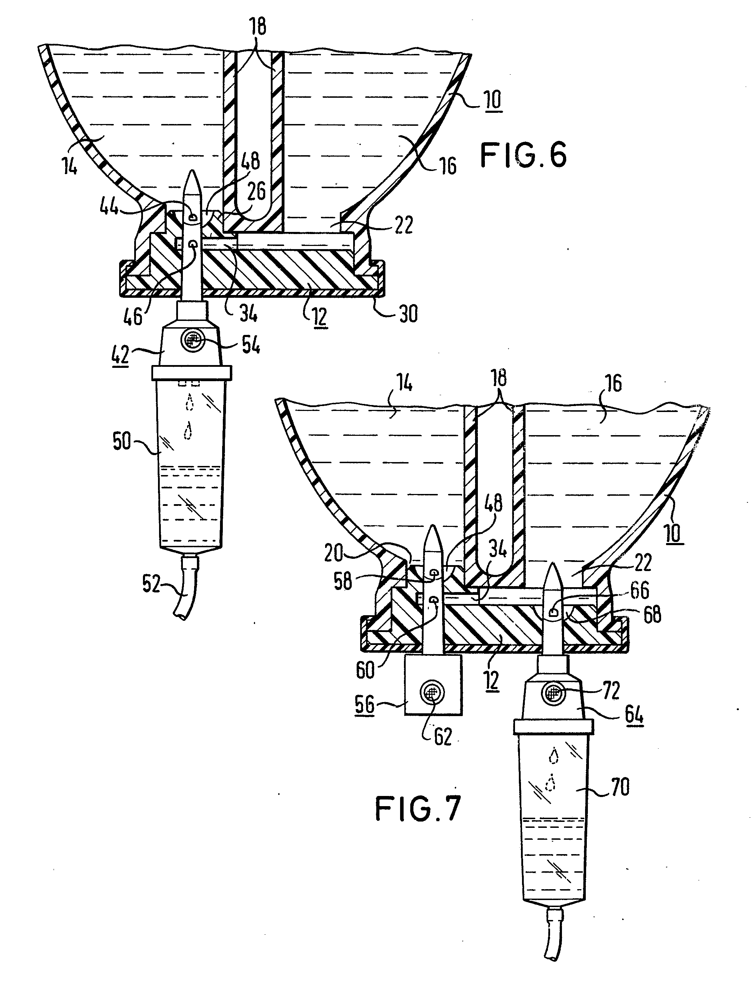

Fig. 6 entspricht weitgehend Fig. 1, zeigt jedoch zusätzlich eine Kanüle 42, die in diesem Falle eine Auslauf-Kanüle zur Entnahme von Infusionslösung aus den Kammern 14 und 16 darstellt. Gemäß Fig. 6 weist die Kanüle 42 im Längsabstand liegende Öffnungen 44 und 46 auf, die mit nicht gezeigten, getrennten Kanälen im Inneren der Kanüle verbunden sind und von denen eine in den Hohlraum 34 und die andere in eine Mulde 48 in dem zapfenförmigen Ansatz 26 mündet. Die beiden nicht gezeigten Auslauf-Kanäle erstrecken sich bis zur Oberseite eines mit der Kanüle integrierten, vorzugsweise durchsichtigen Mischgefäßes 50 und tropfen von dort herab, wie es in Fig. 6 angedeutet ist. Vom Boden des Mischgefäßes 50 geht eine gemeinsame Infusionsleitung 52 aus, durch die die gemischte Lösung verabreicht werden kann.

Zum Ausgleichen des Druckes in den Kammern 14, 16 bei auslaufender Infusionslösung weist die Kanüle 42 außerhalb des Verschlußstopfens 12 ein Luftfilter 54 auf, das über nicht gezeigte Belüftungskanäle mit dem Hohlraum 34 einerseits und der Kammer 14 andererseits in Verbindung steht und Luft in die Kammern einläßt.

Der Behälter 10 und der Verschlußstopfen 12 gemäß Fig. 7 entsprechent vollständig den entsprechenden Teilen der Fig. 6, so daß eine erneute Erläuterung nicht notwendig ist. Im Unterschied zu Fig. 6 ist bei der Ausführungsform der Fig. 7 eine weitere Kanüle 56 vorgesehen, die als Verbindungs-Kanüle zwischen der ersten Kammer 14 und dem Hohlraum 34 dient. Dementsprechend weist die Verbindungs-Kanüle 56 zwei in Längsabstand liegende Öffnungen 58 und 60 auf, die durch einen nicht gezeigten Verbindungs-Kanal miteinander verbunden sind und in die Kammer 14 einerseits sowie den Hohlraum 34 andererseits münden. Ein Luftfilter 62 an der Verbindungs-Kanüle 56 außerhalb des Verschlußstopfens 12 steht mit einem nicht gezeigten Belüftungskanal in Verbindung, der einen Lufteinlaß in die erste Kammer 14 gestattet.

Eine weitere Kanüle 64 in der Form einer Auslauf-Kanüle durchdringt den Verschlußstopfen 12 im Bereich der Öffnung 22 der zweiten Kammer 16 und weist eine Öffnung 66 auf, die in eine Mulde 68 an der Innenseite des Verschlußstopfens mündet. An der Unterseite der Kanüle 64 befindet sich wiederum ein Mischbehälter 70. Ein Luftfilter 72 dient zum Einlassen von Luft in die Kammer 16 über einen nicht gezeigten Belüftungskanal.

Bei dieser Ausführungsform wird zunächst durch die Verbindungs-Kanüle 56 eine Verbindung zwischen der ersten Kammer 14 und dem Hohlraum 34 und damit zugleich mit der zweiten Kammer 16 geschaffen. Die Infusionslösung aus der ersten Kammer 14 gelangt damit an den Boden der zweiten Kammer 16, so daß sich dort ein Gemisch von beiden Infusionslösungen bildet. Dieses Gemisch wird durch die Auslauf-Kanüle 64 abgeführt.

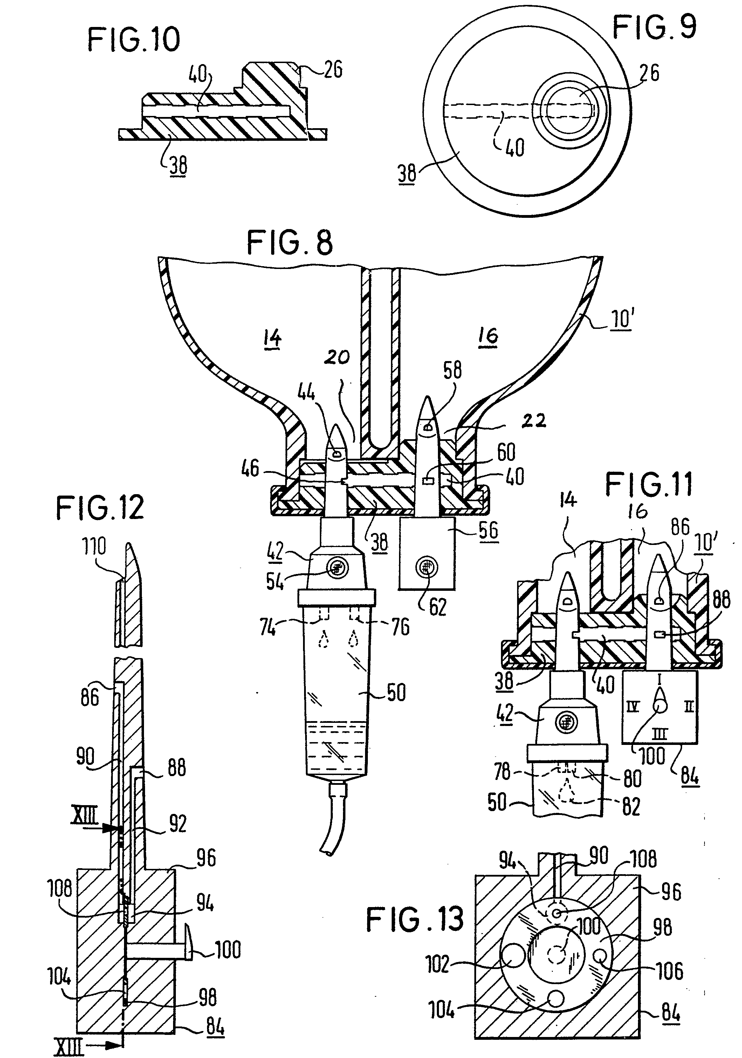

Fig. 8 zeigt einen Behälter 10′ mit einem Verschlußstopfen 38 gemäß Fig. 5, so daß die bereits beschriebenen Teile nicht erneut erläutert werden sollen.

In die Öffnung 22 der zweiten Kammer 16 ist eine Verbindungs-Kanüle 56 eingesetzt, wie sie bereits im Zusammenhang mit Fig. 7 erläutert wurde. Diese Verbindungs-Kanüle weist eine Öffnung 58 innerhalb der Kammer 16 und eine Öffnung 60 innerhalb des Hohlraumes 40 auf, die durch eine nicht gezeigte Verbindungsleitung miteinander in Verbindung stehen. Ein Luftfilter 62 dient zum Einlassen von Luft in die Kammer 16.

Im Bereich der Öffnung 20 der ersten Kammer 14 ist eine Auslauf-Kanüle in den Verschlußstopfen 38 eingestochen, die der Auslauf-Kanüle 42 gemäß Fig. 6 entspricht und daher unter Verwendung entsprechender Bezugsziffern erläutert werden soll. Öffnungen 44 und 46 münden in die erste Kammer 14 einerseits und den bis in den Bereich der Öffnung 22 verlängerten Hohlraum 40 innerhalb des Verschlußstopfens 38 andererseits. Weiterhin ist ein Luftfilter 54 an der Kanüle 42 vorgesehen, das eine Belüftung der ersten Kammer 14 über einen nicht gezeigten Lüftungskanal gestattet.

Von den Öffnungen 44, 46 gehen im Inneren der Kanüle 42 nicht gezeigte Auslauf-Kanäle aus, die innerhalb des Mischgefäßes 50 senkrecht nach unten herabragende Abtropfenden 74, 76 bilden, von denen die Lösungen herabtropfen.

Bei dieser Ausführungsform wird zunächst eine Verbindung zwischen der zweiten Kammer 16 und dem Hohlraum 40 mit Hilfe der Verbindungs-Kanüle 56 hergestellt. Sodann können beide Lösungen gleichzeitig aus der ersten Kammer 14 einerseits und dem Hohlraum 40 andererseits über die Öffnungen 44 und 46 mit Hilfe der Kanüle 42 entnommen werden.

Die Ausführungsform gemäß Fig. 11 entspricht weitgehend denjenigen der Fig. 8 und soll daher nur hinsichtlich bestehender Unterschiede erläutert werden. Zunächst ist erkennbar, daß in diesem Falle die mit 78 und 80 bezeichneten Abtropfenden der Auslaß-Kanäle innerhalb des Mischgefäßes 50 unmittelbar nebeneinander gesetzt sind, so daß sie einen gemeinsamen Tropfen 82 bilden, der bereits eine Mischung beider Lösungen darstellt.

Sodann ist eine Verbindungs-Kanüle 84 gezeigt, die weitgehend der Verbindungs-Kanüle 56 entspricht, jedoch zusätzlich ein Dosier-Ventil enthält. Durch dieses Dosier-Ventil kann der Austritt der Lösung aus der zweiten Kammer 16 in den Hohlraum 40 und damit das Mischungsverhältnis beider Lösungen geregelt werden. Gemäß Fig. 12 sind die Öffnungen 86 und 88, die gemäß Fig. 11 innerhalb der zweiten Kammer 16 bzw. des Hohlraumes 40 liegen, mit Kanal-Abschnitten 90 und 92 verbunden, die innerhalb der Kanüle 84 parallel senkrecht nach unten geführt sind und über einen senkrecht zu den Kanal-Abschnitten 90 und 92 verlaufenden Kanal-Abschnitt 94 innerhalb eines Gehäuses 96 am unteren Ende der Kanüle 84 miteinander in Verbindung stehen.

Innerhalb des Gehäuses 96 ist eine kreisförmige Lochscheibe 98 drehbar gelagert, deren Löcher 102, 104, 106, 108 unterschiedlichen Durchmessers wahlweise in den Kanal-Abschnitt 94 mit Hilfe eines Drehgriffes 100 verschoben werden können. Die Löcher 102, 104, 106, 108 unterschiedlichen Durchmessers sind in Fig. 13 schematisch dargestellt. Gemäß Fig. 13 befindet sich das Loch 108 mit dem kleinsten Durchmesser innerhalb des Kanal-Abschnitts 94, so daß die Lochscheibe in diesem Falle nur den geringsten Durchgang der Lösung gestattet. Selbstverständlich kann auch eine andere Anzahl von Löchern vorgesehen sein.

Fig. 12 läßt im übrigen einen Teilbereich eines Belüftungskanals 110 erkennen, der in nicht gezeigter Weise mit einem beispielsweise in Fig. 8 dargestellten Luftfilter 54 in Verbindung steht.

Die zuvor beschriebenen, unterschiedlichen Ausführungsformen lassen erkennen, daß es sich nur um Beispiele zur Ausführung der Erfindung handelt. Wesentlich ist nur eine zwei Öffnungen aufweisende Kanüle und ein Verschlußstopfen, der es gestattet, die Kanüle mit dem Inhalt beider Kammern mit je einer Öffnung in Verbindung zu bringen. Der Verschlußstopfen muß nicht notwendig für beide Öffnungen in einem Stück ausgebildet sein. Vielmehr können auch getrennte Verschlußstopfen für beide Öffnungen vorgesehen sein, sofern einer der Verschlußstopfen einen inneren Hohlraum aufweist, der in geeigneter Weise, beispielsweise über eine Öffnung der Trennwand zwischen den Kammern mit der anderen Kammer in Verbindung steht.

Ggf. ist es auch möglich, den Verschlußstopfen nicht unmittelbar an den Öffnungen der Kammern, sondern als Ventilsystem am Ende von Zwischenleitungen vorzusehen, die von den Kammern ausgehen.

Im übrigen liegt es auf der Hand, daß auch eine größere Anzahl von Kammern zusammengefaßt werden kann, sofern ein Verschlußstopfen mit Hohlräumen in unterschiedlichen Ebenen und eine Kanüle mit in diesen Ebenen liegenden Öffnungen verwendet werden.

**Ansprüche**

1. Infusionsvorrichtung mit einem eine Öffnung auf der unteren Seite aufweisenden, durch einen Verschlußstopfen (12, 38) verschließbaren Behälter (10, 10') zur Aufnahme einer Infusionslösung und einer den Verschlußstopfen durchdringenden Auslauf-Kanüle (42, 64), dadurch gekennzeichnet, daß der Behälter (10, 10') wenigstens zwei nebeneinander liegende, getrennte Kammern (14, 16) aufweist, daß die Öffnung (20) einer ersten Kammer (14) durch einen Verschlußstopfen (12, 38) mit wenigstens einem inneren Hohlraum (34, 40) verschlossen ist, daß der Hohlraum (34, 40) die Bahn einer Kanüle (42, 56, 64, 84) schneidet und mit einer zweiten Kammer (16) in Verbindung steht und daß diese Kanüle mindestens eine in die erste Kammer (14) und eine in den Hohlraum (34, 40) mündende Öffnung (44, 46, 48, 60, 86, 88) aufweist und als Auslauf-Kanüle (42) ausgebildet ist oder über den Hohlraum (34) mit einer Auslauf-Kanüle (64) in Verbindung steht.

2. Infusionsvorrichtung nach Anspruch 1, dadurch gekennzeichnet, daß die in die erste Kammer (14) eintretende, den Hohlraum (34) durchdringende Kanüle (42) eine Auslauf-Kanüle ist, die vier Kanäle einschließt, von denen zwei Entnahmekanäle sind, die über eine Öffnung (44) bzw. (46) in die erste Kammer (14) bzw. den Hohlraum (34) münden, während jeweils ein Belüftungskanal die erste Kammer bzw. den Hohlraum mit einer nach außerhalb mündenden Lüftungsöffnung verbindet, wobei die Auslauf-Kanüle mit einem Mischgefäß (50) in Verbindung steht, von dem eine Infusionsleitung (52) ausgeht.

3. Infusionsvorrichtung nach Anspruch 2, dadurch gekennzeichnet, daß in der Öffnung (22) der zweiten Kammer (16) ein Verschlußstopfen (38) mit einem inneren Hohlraum (40) vorgesehen ist, der mit dem Hohlraum des Verschlußstopfens der ersten Kammer (14) in Verbindung steht, und daß der Hohlraum (40) des Verschlußstopfens (38) der zweiten Kammer (16) über eine Verbindungs-Kanüle (56, 84) mit der zweiten Kammer (16) verbunden ist, die in die zweite Kammer (16) und den Hohlraum (40) eintretende, durch einen Verbindungskanal verbundene Öffnungen (58, 60, 86, 88) sowie einen in die zweite Kammer (16) eintretenden Belüftungskanal aufweist.

4. Infusionsvorrichtung nach Anspruch 1, dadurch gekennzeichnet, daß die in die erste Kammer (14) eintretende, den Hohlraum (34) durchdringende Kanüle (56) eine Verbindungs-Kanüle (56) ist, deren in die erste Kammer (14) und den Hohlraum (34) mündende Öffnungen (58, 60) durch einen Verbindungskanal verbunden sind und die wenigstens einen von außen in die erste Kammer (14) eintretenden Belüftungskanal enthält, und daß in den Verbindungsbereich zwischen den Hohlraum (34) und der zweiten Kammer (16) eine mit einer Infusionsleitung über ein Mischgefäß (70) verbundene Auslauf-Kanüle (64) eintritt, die einen Kanal mit einer in den Verbindungsbereich mündenden Öffnung (66) und einen Belüftungskanal enthält.

5. Infusionsvorrichtung nach Anspruch 3 oder 4, dadurch gekennzeichnet, daß in den Verbindungskanal der Verbindungs-Kanüle (56, 84) ein Dosierventil (94, 98, 100) eingeschaltet ist.

6. Infusionsvorrichtung nach Anspruch 5, dadurch gekennzeichnet, daß das Dosierventil eine in einer Ebene senkrecht zu einem Abschnitt (94) des Verbindungskanals drehbare Lochscheibe (98) mit Löchern (102, 104, 106, 108) unterschiedlichen Durchmessers umfaßt, die durch Drehen eines Betätigungsgriffes (100) wahlweise in die Bahn des Abschnitts (94) verschiebbar sind.

7. Infusionsvorrichtung nach einem der vorhergehenden Ansprüche, dadurch gekennzeichnet, daß die Öffnungen (20, 22) der Kammern (14, 16) nebeneinander angeordnet und durch einen gemeinsamen Verschlußstopfen (12, 38) verschließbar sind, der mit wenigstens einem zapfenförmigen Ansatz (26) in eine der Öffnungen (20, 22) eintritt.

8. Infusionsvorrichtung nach Anspruch 7, dadurch gekennzeichnet, daß die Öffnungen (20, 22) der Kammern (14, 16) durch einen gemeinsamen, von der Ebene der Öffnungen vorspringenden Rand (24) umgeben sind, der den gemeinsamen Verschlußstopfen (12, 38) aufnimmt.

9. Infusionsvorrichtung nach einem der Ansprüche 2 bis 8, dadurch gekennzeichnet, daß die von den Kammern (14, 16) und/oder Hohlräumen (34, 40) ausgehenden Kanäle an der Oberseite des Mischgefäßes (50, 70) im wesentlichen senkrecht herabragende Abtropfenden (78, 80) aufweisen, die derart nah aneinander angeordnet sind, daß die aus den einzelnen Kanälen zugeführten Infusionslösungen in einem gemeinsamen Tropfen (82) herabfallen.

10. Infusionsvorrichtung nach einem der Ansprüche 2 bis 9, dadurch gekennzeichnet, daß die Belüftungskanäle der Verbindungs-Kanülen (56, 84) und/oder der Auslaufkanülen (42, 64) Luftfilter (54, 62, 72) einschließen.

## Claims

1. An infusion apparatus with a vessel (10, 10') having a lower opening and able to be shut off by a stopper (12, 38), for an infusion solution, and an outlet hollow needle (42, 64) pushed through the stopper, characterized in that the vessel (10, 10') has at least two separate spaces (14, 16) placed side by side, in that the opening (20) of a first space (14) is shut off by the stopper (12, 38) with at least one inner space (34, 40), in that the inner space (34, 40) is in the way of the needle (42, 56, 64, 84) and is joined up with a second space (16), and in that this needle has at least one opening (44, 46, 48, 60, 86, 88) into the first space (14) and into the inner space (34, 40) and is designed as an outlet needle (42) or, by way of inner space (34) is joined up with an outlet needle (64).

2. An infusion apparatus as claimed in claim 1, characterized in that the needle (42) running into the first space (14) through inner space (34) is an outlet needle having four fluidways in it of which two are outlet fluidways opening (at 44 and 46) into the first space (14) and, in the other case, into the inner space (34), one airway being used for joining the first space or, in the other case, the inner space with a venting opening to the outside, the outlet needle being joined up with a mixing vessel (50), with an infusion pipe (52) running from it.

3. An infusion apparatus as claimed in claim 2, characterised in that a stopper (38), having an inner space (40) is placed in the opening (22) of the second space (16) and the inner space (40) is joined up with the inner space of the stopper of the first space (14), and in that the inner space (40) of the stopper (38) for the second space (16) is joined up by way of a connection needle (56, 84) with the second space (16) which has openings (58, 60, 86, 88) into the second space (16) and into the inner space (40), such openings (58, 60, 86, 88) being joined up by a connection, and furthermore having an airing fluidway opening into the second space (16).

4. An infusion apparatus as claimed in claim 1, characterized in that the needle (56) running through the inner space (34) into the first space (14) is a connection needle (56) whose openings (58, 60) into the first space (14) and, in the other case, into the inner space (34) are joined by a connection and which has at least one airway running in from the outside into the first space (14), and in that there is an outlet needle (64) running into the connection part between the inner space (34) and the second space (16), such needle (64) being joined up with an infusion pipe by way of a mixing vessel (70) and having a fluidway, opening (at 66) into the connection part and an airway for airing or venting purposes.

5. An infusion apparatus as claimed in claim 3 or claim 4, characterized in that in the connection of the connection needle (56, 84) there is a rate controlling valve (94, 98, 100).

6. An infusion apparatus as claimed in claim 5, characterized in that the rate controlling valve has a round plate (98) with holes (102, 104, 106, 108), such plate being able to be turned in a plane normal to one part (94) of the connection, the holes, having different diameters, being able to be moved into the path of part (94) as may be desired by turning a handle (100).

7. An infusion apparatus as claimed in anyone of claims 1 to 6, characterized in that the openings (20, 22) of the space (14, 16) are placed side by side and may be shut off by a common stopper (12, 38), such stopper having at least one pin-like head (26) which is put into one of the openings (20, 22).

8. An infusion apparatus as claimed in claim 7, characterized in that the openings (20, 22) of the spaces (14, 16) have a common edgepiece (24), running out from the plane of the openings, round them taking up the common stopper (12, 38).

9. An infusion apparatus as claimed in anyone of claims 2 to 8, characterized in that the fluidways running out from the spaces (14, 16) and/or the inner spaces (34, 40) on the top side of the mixing vessel (50, 70) have drop-off ends (78, 80) which are generally upright and placed so near each other that the infusion solutions coming from the separate fluidways are dropped down as a single drop (82).

10. An infusion apparatus as claimed in anyone of claims 2 to 9, characterized in that the airing fluidways of the connection needles (56, 84) and/or of the outlet needles (42, 64) have air filters (54, 62, 72) in them.

## Revendications

1. Dispositif de perfusion comportant un récipient (10, 10'), présentant une ouverture sur le côté inférieur, et susceptible d'être fermé par un bouchon de fermeture (12, 38), pour recevoir une solution de perfusion, et une canule de sortie (42, 64) traversant le bouchon de fermeture, caractérisé par le fait que le récipient (10, 10') présente au moins deux chambres (14, 16) séparées et disposées côte à côte, que l'ouverture (20) d'une première chambre (14) est fermée par un bouchon de fermeture (12, 38) comportant au moins une cavité interne (34, 40), que la cavité (34, 40) coupe le trajet d'une canule (42, 56, 64, 84) et est en communication avec une deuxième chambre (16) et que cette canule présente des ouvertures (44, 46, 48, 60, 86, 88) dont une au moins débouche dans la première chambre (14) et une au moins dans la cavité (34, 40), et est réalisée sous la forme d'une canule de sortie (42) ou est en communication par la cavité (34) avec une canule de sortie (64).

2. Dispositif de perfusion selon la revendication 1, caractérisé par le fait que la canule (42) pénétrant dans la première chambre (14) et traversant la cavité (34) est une canule de sortie qui comporte quatre canaux dont deux sont des canaux de soutirage qui débouchent par une ouverture (44) et respectivement (46) dans la première chambre (14) et respectivement la cavité (34), tandis qu'à chaque fois un canal d'aération relie la première chambre et respectivement la cavité avec une ouverture d'aération débouchant vers l'extérieur, la canule de sortie étant en communication avec un récipient de mélange (50) dont sort un conduit de perfusion (52).

3. Dispositif de perfusion selon la revendication 2, caractérisé par le fait que dans l'ouverture (22) de la deuxième chambre (16) est prévu un bouchon de fermeture (38) avec une cavité interne (40) qui est en communication avec la cavité du bouchon de fermeture de la première chambre (14), et que la cavité (40) du bouchon de fermeture (38) de la deuxième chambre (16) est reliée à la deuxième chambre (16) par une canule de liaison (56, 84) qui présente des ouvertures (58, 60, 86, 88) pénétrant dans la deuxième chambre (16) et la cavité (40), reliées par un canal de liaison, ainsi qu'un canal d'aération pénétrant dans la deuxième chambre (16).

4. Dispositif de perfusion selon la revendication 1, caractérisé par le fait que la canule (56) pénétrant dans la première chambre (14), traversant la cavité (34), est une canule de liaison (56) dont les ouvertures (58, 60) débouchant dans la première chambre (14) et la cavité (34) sont reliées par un canal de liaison, et qui contient au moins un canal d'aération pénétrant de l'extérieur dans la première chambre (14), et que dans la zone de liaison entre la cavité (34) et la deuxième chambre (16) pénètre une canule de sortie (64) reliée à un conduit de perfusion par un récipient de mélange (70), et contenant un canal avec une ouverture (66) débouchant dans la zone de liaison et un canal d'aération.

5. Dispositif de perfusion selon la revendication 3 ou 4, caractérisé par le fait que dans le canal de liaison de la canule de liaison (56, 84) est mise en place une soupape doseuse (94, 98, 100).

6. Dispositif de perfusion selon la revendication 5, caractérisé par le fait que la soupape doseuse comprend un disque percé (98) avec des trous (102, 104, 106, 108) de diamètres différents, disposé dans un plan perpendiculaire à un tronçon (94) du canal de liaison, lesdits trous étant déplaçables par rotation d'un bouton d'actionnement (100) à volonté dans le trajet du tronçon (94).

7. Dispositif de perfusion selon l'une des revendications précédentes, caractérisé par le fait que les ouvertures (20, 22) des chambres (14, 16) sont disposées les unes à côté des autres et sont susceptibles d'être fermées par un bouchon de fermeture commun (12, 38) qui pénètre par au moins un prolongement (26), en forme de tenon, dans l'une des ouvertures (20, 22).

8. Dispositif de perfusion selon la revendication 7, caractérisé par le fait que les ouvertures (20, 22) des chambres (14, 16) sont entourées par un bord (24) commun, faisant saillie du plan des ouvertures, et qui reçoit le bouchon de fermeture commun (12, 38).

9. Dispositif de perfusion selon l'une des revendications 2 à 8, caractérisé par le fait que les canaux sortant des chambres (14, 16) et/ou des cavités (34, 40) présentent, à la partie supérieure du récipient de mélange (50, 70), des extrémités d'égouttage (78, 80) faisant saillie sensiblement verticalement vers le bas et qui sont disposées à proximité l'une de l'autre de telle sorte que les solutions de perfusion amenées par les canaux séparés tombent en une goutte commune (82).

10. Dispositif de perfusion selon l'une des revendications 2 à 9, caractérisé par le fait que les canaux d'aération des canules de liaison (56, 84) et/ou des canules de sortie (42, 64) comprenant des filtres à air (54, 62, 72).

0 012 445

FIG.2

FIG.3

FIG.1

FIG.4

18'    18'

10'

20    22

24'

FIG.5

10'

18'    18'

14    16

20    26    40    22

30    24'    38

FIG.6

FIG.7

0 012 445

FIG.10

FIG.9

FIG.8

FIG.12

FIG.11

FIG.13

4